# EUROPEAN PATENT APPLICATION

(11) **EP 4 410 242 A1**
(43) Date of publication of application: **07.08.2024**
(21) Application number: 22926842.0
(22) Date of filing: 01.12.2022
(51) Int. Cl.: A61F 2/24

(54) **ANCHORING DEVICES OF ARTIFICIAL HEART VALVE AND ARTIFICIAL HEART VALVE SYSTEM**

(30) Priority: 18.02.2022 CN 202210152752
(71) Applicant: Shanghai Trulive Medtech Co., Ltd, Shanghai 201206 (CN); Jiangsu Trulive Medtech Co., Ltd., Nantong, Jiangsu 226400 (CN)
(72) Inventor: HE, Dong, Shanghai 201206 (CN); MA, Kangling, Shanghai 201206 (CN); LIU, Xiang, Shanghai 201206 (CN); WEI, Yongqiang, Shanghai 201206 (CN)
(74) Representative: Nordmeyer, Philipp Werner
(86) International application number: PCT/CN2022/135762
(87) International publication number: WO 2023/155538

(57) **Abstract**

An anchoring device (100, 200, 300) for prosthetic heart valve (400) and a prosthetic heart valve system. The prosthetic heart valve system includes an anchoring device (100, 200, 300) and a prosthetic heart valve (400). The anchoring device (100, 200, 300) includes an anchoring section (110, 210, 310) and a supporting section (120, 220, 320) that are axially connected to each other. The anchoring section (110, 210, 310) has an annular first cavity (111, 211, 311) configured to accommodate a prosthetic heart valve (400). The supporting section (120, 220, 320) is arranged on one side of the anchoring section (110, 210, 310) along the axial direction thereof. The supporting section (120, 220, 320) includes at least two elastic wires (121, 221, 321) arranged sequentially on the circumference of the anchoring section (110, 210, 310). Each elastic wire (121, 221, 321) has an end connected to the anchoring section (110, 210, 310), and a further end extending in a direction away from the anchoring section (110, 210, 310). The anchoring device (100, 200, 300) can utilize the structure of the predetermined chamber to anchor the prosthetic heart valve (400) without crossing the native valve annulus during implantation, thereby effectively preventing the occurrence of paravalvular leakage after the prosthetic heart valve (400) is implanted.

## Description

### TECHNICAL FIELD

The present application relates to the technical field of medical devices and, more specifically, to an anchoring device for prosthetic heart valve and a prosthetic heart valve system.

### BACKGROUND

Transcatheter mitral valve replacement (TMVR) adopts a catheter intervention method to compress the prosthetic heart valve into the delivery system outside the body, then deliver it to the human mitral valve annulus along the vascular path or through the cardiac apex, and release and fix the prosthetic heart valve at the mitral valve annulus to replace the native valve. Compared with surgery, mitral valve replacement does not require extracorporeal circulation auxiliary devices, has less trauma, and the patient recovers quickly. The patient's hemodynamic index can be significantly improved after surgery. Compared with the apical approach, the implantation via the atrial septal approach through the femoral vein is more convenient. It is less invasive and has a wider audience.

Although technology for mitral valve replacement has developed rapidly, the anchoring of prosthetic heart valves still has certain limitations. According to the traditional anchoring method, the prosthetic heart valve is fixed mainly by designing anchoring stabs configured to grasp the valve leaflets, or over-sizing the main body of the prosthetic heart valve. However, these two anchoring methods can easily cause damage to the native valve leaflets or compression of the native valve annulus tissue, thus adversely affecting the patient's recovery.

Prosthetic heart valves in the prior art also adopt a design in which the anchoring device is separated from the prosthetic heart valve. For example, the prosthetic heart valve is composed of a multi-turn docking coil and a cylindrical valve body. This design of separating the anchoring device and the valve body can effectively avoid the compression of the native valve annulus and is not easy to damage the valve leaflets. At the same time, it reduces the size of each part of the delivery system, which is more conducive to the movement of the delivery system in the body. However, this docking coil includes an atrial coil segment and a ventricular coil segment. The connection between the atrial coil segment and the ventricular coil segment will pass through the native valve annulus, thus there is a risk of paravalvular leakage at the native valve annulus. In addition, since the release process of the docking coil is relatively complicated and there is a high requirement in positioning the docking coil. This will increase the difficulty of the prosthetic heart valve implantation operation and place high requirements on the operator's proficiency. At the same time, the long duration of surgery also adversely affects the patient's health.

### SUMMARY OF THE INVENTION

In order to solve the technical problems existing in the prior art, an object of the present invention is to provide an anchoring device for prosthetic heart valve and a prosthetic heart valve system. The anchoring device can utilize the structure of the predetermined chamber to anchor the prosthetic heart valve without crossing the native valve annulus during implantation, thereby effectively preventing the occurrence of paravalvular leakage after the prosthetic heart valve is implanted.

In order to achieve at least one of the above objects, the present invention provides an anchoring device for prosthetic heart valve, comprising an anchoring section and a supporting section that are axially connected to each other; wherein the anchoring section has an annular first cavity configured to accommodate a prosthetic heart valve, and the anchoring section is configured to self-adapt to an outer contour of the prosthetic heart valve when being deformed by a force so as to exert anchoring force on the prosthetic heart valve;
the supporting section is arranged on one side of the anchoring section along an axial direction thereof, and includes at least two elastic wires arranged sequentially on a circumference of the anchoring section; each of the elastic wires has an end connected to the anchoring section, and has a further end extending in a direction away from the anchoring section;
the anchoring device is configured such that the anchoring section is configured to be arranged at annulus of a predetermined object, and the supporting section is configured to be arranged in a predetermined chamber of the predetermined object and can be fixed in the predetermined chamber by elastic force.

Optionally, in a radial direction of the anchoring device, a maximum outer diameter of the supporting section after expansion is greater than an inner diameter of a contact portion of the predetermined chamber to the supporting section, and/or, in an axial direction of the anchoring device, an axial height of the supporting section after expansion is greater than an axial height of the predetermined chamber.

Optionally, a partial contour of the elastic wire along an extension direction thereof is curved.

Optionally, a partial contour of the elastic wire along the extension direction thereof is arc-shaped or wavy.

Optionally, each of the elastic wires comprises an avoidance section, and wherein the avoidance sections on all the elastic wires jointly define a second cavity configured to accommodate at least part of the prosthetic heart valve; after the anchoring device is expanded, an inner diameter of the second cavity is larger than an inner diameter of the first cavity.

Optionally, the anchoring device for prosthetic heart valve further comprises a fixing part connected to the further end of one or more of the elastic wires, and wherein the fixing part is arranged in the predetermined chamber.

Optionally, the fixing part is a fixing ring, and an inner diameter of the fixing ring is smaller than an inner diameter of the first cavity after the anchoring device is expanded.

Optionally, all the elastic wires are evenly distributed on the circumference of the anchoring section.

Optionally, the anchoring section is structured as a coil formed by winding wire material, and the coil has at least one turn.

In order to achieve the above objects, the present invention provides a prosthetic heart valve system, comprising a prosthetic heart valve and the above anchoring device for prosthetic heart valve, wherein the prosthetic heart valve is configured to be accommodated in the anchoring device and fixed in the predetermined object by the anchoring device.

According to the anchoring device and the prosthetic heart valve system in the present invention, the prosthetic heart valve can be fixed at the heart valve annulus by the anchoring device. In specific implementation, the anchoring section of the anchoring device is provided at the native valve annulus, and the anchoring section is fixed by virtue of the anchoring of the supporting section in the atrium or ventricle. In this way, since the supporting section is only provided on one side of the anchoring section along the axial direction, the supporting section does not need to cross the native valve annulus, thereby effectively preventing the occurrence of paravalvular leakage. In addition, this also avoids damage to the native valve leaflets or compression of the native valve annulus tissue. The operation is safe and reliable, and is beneficial to the patient's postoperative recovery.

The anchoring device for prosthetic heart valve provided by the present invention achieves anchoring only through the interference fit between the supporting section and the atrium or ventricle due to the elasticity of the supporting section, and the prosthetic heart valve is connected to the anchoring section by the interference fit between the first cavity of the anchoring section and the prosthetic heart valve, which results in a simple structure. When the anchoring device is released at any position in the atrium or ventricle during the implantation, the anchoring section can be moved automatically to the desired position, which can simplify the implantation process of the anchoring device, reduce the difficulty of implanting the anchoring device and improve the reliability and convenience of prosthetic heart valve surgery.

### BRIEF DESCRIPTION OF THE DRAWINGS

Fig. 1 is a schematic diagram showing the structure of an anchoring device according to Embodiment 1 of the present invention;
Fig. 2 is a schematic diagram showing the structure of an anchoring device according to a preferable example in Embodiment 2 of the present invention;
Fig. 3 is a schematic diagram showing the structure of an anchoring device according to another preferable example in Embodiment 2 of the present invention;
Fig. 4 is a schematic diagram showing the structure of an anchoring device according to Embodiment 3 of the present invention;
Fig. 5 shows a usage scenario of the anchoring device according to Embodiment 3 of the present invention.

In the drawings:
anchoring devices 100, 200, 300; anchoring sections 110, 210, 310; first cavities 111, 211, 311; supporting sections 120, 220, 320; elastic wires 121, 221, 321; avoidance sections 222, 322; fixing parts 130, 230; prosthetic heart valve 400; outflow section 410; annulus section 420; flange section 430.

### DETAILED DESCRIPTION

The present invention will be described in greater detail below with reference to specific embodiments which are to be read in conjunction with the accompanying drawings. Advantages and features of the present invention will be apparent from the description below. It is noted that the figures are provided in a very simplified form not necessarily presented to scale, with their only intention to facilitate convenience and clarity in explaining the disclosed embodiments.

The orientation or positional relationship indicated by the terms "center", "lengthwise", "crosswise", "length", "width", "thickness", "above", "below", "front", "back", "left", "right", "vertical", "horizontal", "top", "bottom", "inside", "outside", "clockwise", "counterclockwise", "axial", "radial", "circumferential" is based on the orientation or positional relationship shown in the drawings, which is only for the convenience of describing the present invention and simplifying the description, and does not indicate or imply that the device or element referred to must have a specific orientation or be constructed in a specific orientation, and therefore cannot be construed as limitations of the present invention.

In the present invention, unless otherwise expressly stipulated and limited, the terms "install", "connect", "fix" and other terms should be understood in a broad sense. For example, it can be a fixed connection, a detachable connection, or an integrated connection. It can be a mechanical connection, an electrical connection or communication with each other. It can be a direct connection, or it can be connected through an intermediate medium. It can be an internal connection between two elements or an interactive relationship between two elements, unless otherwise specified limited. For those of ordinary skill in the art, the specific meanings of the above terms in the present invention can be understood according to specific circumstances. In this article, the term "radial" refers to the direction parallel to the native valve annulus after the anchoring device is implanted in the heart, that is, the radial direction of the native valve annulus. The term "axial" refers to the direction perpendicular to the native valve annulus after the anchoring device is implanted into the heart, that is, the axial direction of the native valve annulus.

The core idea of the present invention is to provide an anchoring device for prosthetic heart valve and a prosthetic heart valve system. The prosthetic heart valve can be firmly anchored by the anchoring device at the native valve annulus in the heart, for example at the aortic valve, tricuspid valve or mitral valve, thereby replacing the heart valve (aortic, tricuspid, or mitral valve). It is to be understood that the heart contains four chambers, namely the left atrium (LA), the right ventricle (RV), the right atrium (RA) and the left ventricle (LV). Throughout the cardiac cycle, the pumping action of the left and right sides of the heart generally occurs simultaneously. The membrane that separates the atria from the ventricles is called an atrioventricular valve. Each atrium is connected to the corresponding atrial valve through the atrium vestibule. The atrioventricular valve acts as a one-way valve, ensuring the normal flow of blood in the cardiac chambers. The atrioventricular valve between the left atrium and the left ventricle is the mitral valve, and the atrioventricular valve between the right atrium and the right ventricle is the tricuspid valve. The pulmonary valve directs blood flow to the pulmonary artery and to the lungs, and then blood flows through the pulmonary veins to the left atrium. The aortic valve guides blood to flow through the aorta and throughout the body.

During ventricular filling (diastole), the aortic and pulmonic valves close to prevent blood in the arteries from flowing back into the ventricles; at the same time, the mitral and tricuspid valves open to allow blood to pass from the atria into the corresponding ventricles. During the ventricular systole (emptying) phase, the mitral and tricuspid valves close to prevent blood from entering the corresponding ventricles from the atria; at the same time, the aortic and pulmonic valves open to allow blood to be pumped from the ventricles and reach the whole body and lungs through the aorta and pulmonary arteries; then the left atrium and right atrium relax, allowing peripheral blood to return to the left atrium and right atrium.

When there is a problem with the atrioventricular valve, it often causes the atrioventricular valve to fail to close properly. The atrioventricular valve generally includes annulus, leaflets, chordae tendineae and supporting structures. The mitral valve has two leaflets, and the tricuspid valve has three leaflets. The contact between the leaflets can close or seal the mitral valve or tricuspid valve, thereby preventing blood from circulation between the atria and the ventricle during ventricular systole. An incomplete seal between the leaflets of the mitral and tricuspid valves is called valvular insufficiency or malcoaptation, which allows blood in the ventricle to pass through the gap between the mitral or tricuspid valves during ventricular systole, and flow back to the corresponding atrium, which often causes the patient's heart failure, reduced blood flow, lowered blood pressure and reduced oxygen content of the blood reaching various tissues of the body. Atrioventricular valve insufficiency may also cause blood to flow back from the left atrium to the pulmonary veins, thus causing congestion in the lungs; severe atrioventricular valve insufficiency may lead to permanent disability or death if left untreated.

As mentioned in the background art, traditional prosthetic heart valves have problems such as damage to the native valve leaflets or compression of the native valve annulus tissue during anchoring, as well as the risk of paravalvular leakage at the native valve annulus, and complicated surgical operations.

In order to solve at least one technical problem existing in the prior art, the present invention improves the anchoring device for prosthetic heart valve. Specifically, the anchoring device includes an anchoring section and a supporting section that are axially connected to each other. The anchoring section has an annular first cavity configured to accommodate the prosthetic heart valve, and is used to self-adapt to the outer contour of the prosthetic heart valve when the anchoring section is deformed by force, so as to apply anchoring force to the prosthetic heart valve. The supporting section is arranged on one side of the anchoring section along the axial direction thereof. The supporting section includes at least two elastic wires arranged sequentially on the circumference of the anchoring section. One end of each elastic wire is connected to the anchoring section, and the other end extends away from the anchoring section. The anchoring device is configured such that the anchoring section is configured to be disposed at the valve annulus of a predetermined object. The supporting section is configured to be disposed in a predetermined chamber of the predetermined object and can be fixed in the predetermined chamber by elastic force. Such a configuration enables the anchoring device to utilize the structure of a predetermined chamber such as the atrium or ventricle to anchor the prosthetic heart valve. This anchoring method not only avoids damage to the native valve leaflets or compression of the native valve annulus tissue, but also prevents damage to the native valve annulus tissue during implantation. The supporting section does not need to cross the native valve annulus, which can effectively prevent the occurrence of paravalvular leakage, while also simplifying the surgical operation process and reducing the difficulty of the surgical operation. It should be understood that the predetermined object refers to a subject who needs to be implanted with a prosthetic heart valve, and the predetermined object generally refers to the human heart. The predetermined chamber refers to a chamber in the heart that can accommodate and fix the supporting section. The predetermined chamber may be an atrium or a ventricle, preferably an atrium.

The present invention will be described in greater detail below with reference to specific embodiments which are to be read in conjunction with the accompanying drawings. The following embodiments and features of the embodiments may be complemented or combined mutually unless there is any conflict.

### EMBODIMENT 1

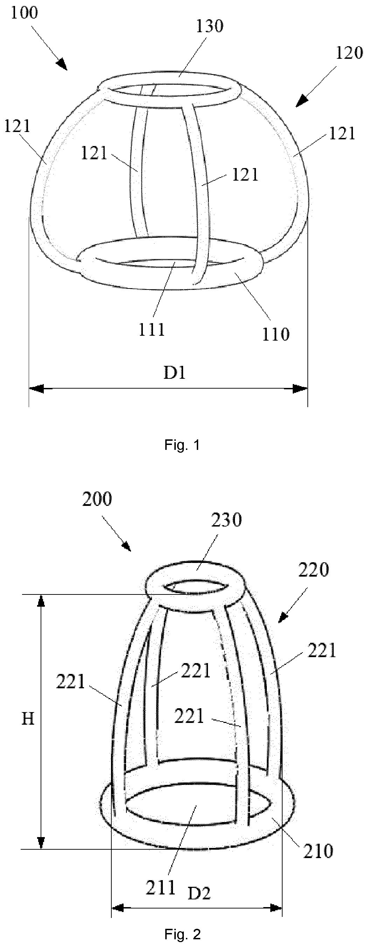

As shown in Fig. 1, the preferred Embodiment 1 of the present invention provides an anchoring device 100 for prosthetic heart valve. The anchoring device 100 includes an anchoring section 110 and a supporting section 120 that are axially connected to each other. The supporting section 120 is configured to limit the position of the anchoring section 110. Specifically, the supporting section 120 is configured to support the anchoring section 110 mainly by virtue of native myocardial tissue such as the atrial wall or ventricular wall to prevent the anchoring section 110 from being displaced during the cardiac cycle. The supporting section 120 is only provided on one side of the anchoring section 110 along the axial direction thereof to avoid crossing the native valve annulus. The supporting section 120 includes at least two elastic wires 121 arranged sequentially along the circumference of the anchoring section 110. One end of each elastic wire 121 is connected to the anchoring section 110, and the other end extends in a direction away from the anchoring section 110. Specifically, the extension direction of the elastic wire 121 is parallel to the axis direction of the anchoring section 110, or forms an included angle less than or equal to 90° with the axis of the anchoring section 110. The axial direction of the anchoring section 110 here refers to the direction from the anchoring section 110 to the supporting section 120.

The anchoring section 110 has an annular first cavity 111 capable of accommodating a prosthetic heart valve. When the anchoring section 110 is deformed by force, it self-adapts to the outer contour of the prosthetic heart valve to apply anchoring force to the prosthetic heart valve. After the force is applied, the prosthetic heart valve and the anchoring section 110 are fixed to each other. Specifically, when the prosthetic heart valve is released in the anchoring device 100, the anchoring section 110 expands after receiving the force resulting from the release of the prosthetic heart valve, causing the anchoring section 110 to undergo elastic and/or plastic deformation. When the anchoring section 110 deforms, it self-adapts to the outer contour of the prosthetic heart valve to apply anchoring force to the prosthetic heart valve, so that the anchoring section 110 is sealed and fixedly connected to the prosthetic heart valve.

The preferred Embodiment 1 of the present invention also provides a prosthetic heart valve system, which includes a prosthetic heart valve and an anchoring device 100. The prosthetic heart valve is configured to be accommodated in the anchoring device 100, so that the anchoring device 100 provides the anchoring force required for anchoring the prosthetic heart valve in the heart. The anchoring device 100 provided by the present invention can be implanted into the body and fixed before the prosthetic heart valve is implanted, thereby providing the anchoring force required for anchoring the prosthetic heart valve in advance.

In more detail, when the anchoring device 100 is implanted in the heart and released, the anchoring section 110 is located at the native valve annulus, and the supporting section 120 is released in the atrium or ventricle, and the supporting section 120 is configured to apply elastic force to the inner wall of the atrium or ventricle so that the supporting section 120 is anchored in the atrium or ventricle. Therefore, the anchoring section 110 is anchored to the native valve annulus. With this arrangement, on one hand, the supporting section 120 is only provided on one side of the anchoring section 110, so that the supporting section 120 does not need to be disposed across the native valve annulus, thereby effectively preventing the occurrence of paravalvular leakage. On another hand, this anchoring method also avoids damage to the native valve leaflets or compression of the native valve annulus tissue, and simplifies the surgical process and reduces the difficulty of the surgical operation.

It should also be understood that the anchoring device 100 provided by the present invention has basically no impact on the movement of the native valve leaflets during the release process. That is, the implantation process of the anchoring device 100 does not hinder the realization of the basic functions of the native valve leaflets. This can ensure that during the implantation process of the anchoring device 100, the heart can always perform periodic motion normally without using an extracorporeal lung machine or stopping the heart, thereby reducing the difficulty of the operation, providing the operator with more time to adjust the position of the anchoring device 100 and better preparation for the implantation of prosthetic heart valve, which greatly reduces the patient's pain and improves the success rate of the operation. In addition, the anchoring device 100 of the present invention does not require anchoring stabs, nor does it need to be fixed by over-sizing the prosthetic heart valve. Therefore, it will not cause damage to the native valve leaflets or pressure on the native valve annulus tissue, and the surgical damage is small, which is more beneficial to the postoperative recovery of patient.

It should be understood that the elastic wire 121 is made of elastic material, so that the supporting section 120 can elastically deform in the radial and/or axial direction of the anchoring device 100. When the supporting section 120 is released in the atrium or ventricle, the supporting section 120 is elastically deformed, and thus the supporting section 120 can exert elastic force on the inner wall of the atrium or ventricle, so that the supporting section 120 is connected firmly to the atrium or ventricle under the action of the elastic force, thereby ensuring that the anchoring section 110 can be positioned at the desired site during the cardiac cycle.

Preferably, the anchoring device 100 is fixed in the atrium, such as the right atrium or the left atrium. In a specific embodiment, the anchoring device 100 may enter the atrium through the interatrial septum or the apex of the heart, and release the anchoring section 110 and the supporting section 120 in the atrium. After release, the anchoring section 110 can naturally displace to the native valve annulus under the action of the expanding force of the supporting section 120. The supporting section 120 can exert elastic force on the inner wall of the atrium after release, so that the anchoring section 110 is anchored at the native valve annulus.

The present application does not limit the elastic material used to prepare the supporting section 120, as long as it is a metal material or polymer material that can produce elastic deformation. For example, the material of the supporting section 120 may be one or a combination of stainless steel, titanium alloy, and nickel-titanium alloy. The material of the supporting section 120 may be a polymer material, such as nylon material. The supporting section 120 may be an integrally formed structure, for example, a plurality of elastic wires 121 are formed by cutting. The supporting section 120 may be formed by integrating separate structures, that is, the elastic wires 121 are processed and manufactured separately and then connected to the anchoring section 110 respectively. The materials of the plurality of elastic wires 121 may be the same or different, and each elastic wire 121 may be made of one or more combinations of materials. For example, different types of material sections may be connected to each other to form the elastic wire 121. The elastic wire 121 can have a solid or hollow structure, as long as it can provide support that is strong enough. The cross-sectional shape of the elastic wire 121 is not limited, such as circular, annular or rectangular, or the elastic wire 121 can also be designed with variable diameter or variable cross-section, that is, the cross-section of a single elastic wire 121 at different positions is different in size or shape, so as to meet the design requirements of the anchoring device 100. In order to increase the friction when the elastic wire 121 is anchored, that is, to further increase the anchoring effect of the supporting section 120 in the heart, a covering layer with a high friction coefficient can be coated on the surface of the elastic wire 121. The covering layer with high friction coefficient may be made of one or a combination of polyethylene, polytetrafluoroethylene, and PET materials.

The number of elastic wires 121 is not limited in this application. Preferably, all elastic wires 121 are evenly distributed on the circumference of the anchoring section 110. The even distribution here can be understood that the elastic wires 121 are distributed at an equal spacing. This allows the anchoring device 100 to have a simple structure, smaller size, and better anchoring effect. When the anchoring device 100 is implanted and released at any position in in the atrium or ventricle, the anchoring section 110 can be automatically moved to the desired position, thereby simplifying the implantation process of the anchoring device and reducing the difficulty of implanting the anchoring device. Therefore, the reliability and convenience of prosthetic heart valve surgery can be improved.

Continuing to refer to Fig. 1, in this embodiment, the supporting section 120 includes four elastic wires 121 evenly distributed on the circumference of the anchoring section 110. Of course, in other embodiments, the number of elastic wires 121 in the supporting section 120 may be 2, 3, 5, 6 or more.

Preferably, the anchoring section 110 is structured as a coil formed by winding a single wire material, and the coil is at least a part of a ring. In one embodiment, the anchoring section 110 may be formed by winding a single wire material. In another embodiment, the anchoring section 110 may be formed by connecting wire segments of two or more materials. For example, wires of different types of materials may be connected to each other to form an anchoring ring. In a specific embodiment, as shown in Fig. 1, the anchoring section 110 has a single annular structure to form an anchoring ring. This can facilitate the implantation of the anchoring section 110 at the native valve annulus, and also facilitate the distribution of the elastic wire 121 on the circumference of the anchoring section 110. In addition to the single annular structure shown in the figure, the anchoring section 110 can also be composed of a plurality of coaxial annular structures fixedly connected to each other. Specifically, the anchoring section 110 may include a plurality of parallel anchoring rings distributed axially connected in sequence. The diameters of the plurality of anchoring rings may be same or different, preferably different, to better adapt to the contour of the implanted heart valve. In another specific embodiment, the anchoring section 110 is a spiral coil. Generally, the number of turns of the spiral coil is 1-5, and the number of turns refers to the winding circles of the spiral coil. Preferably, there are 1-2 turns, so that the anchoring section 110 can be easily elastically deformed when the prosthetic heart valve is implanted and expanded.

This application does not limit the material used to prepare the anchoring section 110. Preferably, the anchoring section 110 is made of elastic material. The anchoring section 110 can at least be elastically deformed in the radial direction of the anchoring device 100, so that the anchoring section 110 can better fixed to and sealed by the prosthetic heart valve. Therefore, the occurrence of paravalvular leakage can be effectively prevented. This application does not limit the elastic material used to prepare the anchoring section 110, as long as it is a metal material or polymer material that can produce elastic deformation. In the embodiment of the present invention, the material of the anchoring section 110 can be one or a combination of stainless steel, titanium alloy, and nickel-titanium alloy. The material of the anchoring section 110 can also be a polymer material, such as nylon material, polyethylene, polyurethane, silicone.

This application does not limit the cross-sectional shape of the wire material used to prepare the anchoring section 110. The cross-sectional shape of the wire material may be circular, annular or rectangular, or the wire material may be designed with variable diameter or variable cross-section, that is, the cross-section of a single wire material at different positions is different in size or shape so as to meet the design requirements of the anchoring device 100. In order to increase the friction for anchoring the anchoring section 110, that is, to further increase the anchoring effect of the anchoring section 110 in the heart, a covering layer with a high friction coefficient can be coated on the surface of the wire material used to prepare the anchoring section 110. The covering layer with high friction coefficient may be made of one or a combination of polyethylene, polytetrafluoroethylene, and PET materials.

In an example, different wire materials can be used to form the anchoring section 110 and the supporting section 120 respectively, and the anchoring section 110 and the supporting section 120 are connected to each other to form the anchoring device 100. That is, the anchoring section 110 and the supporting section 120 are formed separately and then connected to each other. In other examples, a single wire material may be wound successively to form the anchoring section 110 and the supporting section 120 to form the integrated anchoring device 100.

Referring to Fig. 1, in the radial direction of the anchoring device 100, the maximum outer diameter D1 of the expanded supporting section 120 is larger than the inner diameter of the contact portion of the atrium or ventricle with the supporting section 120. This arrangement allows the supporting section 120 to be anchored in an interference fit with the atrium or ventricle in the radial direction, so that the supporting section 120 can be more firmly connected to the atrium or ventricle.

This application does not limit the cross-sectional shape of the expanded supporting section 120, as long as it is ensured that the outer diameter of at least part of the expanded cross-section can form an interference fit with the atrium or ventricle. Preferably, the partial outer contour of the expanded supporting section 120 matches the shape of the contact portion to increase the contact area and improve the anchoring force. For example, the shape of the outer contour of the expanded supporting section 120 can be spherical or spherical-like, so that the anchoring effect is better. For example, the outer contour of the expanded supporting section 120 can also be in other shapes, such as an elliptical shape or a cylindrical shape.

It should also be understood that when the docking coil of the existing anchoring device is released, the docking coil must release the atrial coil segment or the ventricular coil segment from the junctional leaflets, and the coil plane must be parallel to the native valve annulus during the release process. The released atrial coil segment or ventricular coil segment needs to surround all native valve leaflets. This process takes a long time and requires high operating proficiency of the operator. This not only prolongs the patient's operation time, which brings greater pain to the patient, but also very easy to cause a failure in subsequent surgeries due to inaccurate placement of the docking coil. In this application, the elastic wires 121 of the supporting section 120 are evenly distributed on the circumference of the anchoring section 110, so that the anchoring device 100 can be released at any position in the predetermined chamber without the need to find a special angle and orientation to release the supporting section 120. At this time, the anchoring device 100 can move to the position where it needs to be implanted by itself. Specifically, after the anchoring section 110 is released, it can automatically move to the native valve annulus, and after the elastic wires 121 on the supporting section 120 are released and expanded completely, the symmetrically arranged elastic wires 121 can automatically adjust the position thereof until each elastic wire 121 can provide the same elastic force to the predetermined chamber. After the anchoring device 100 is released in the predetermined chamber, the anchoring section 110 and the supporting section 120 in the anchoring device 100 can automatically move to the desired position. In this way, it can simplify the difficulty of implanting the anchoring device 100, greatly improve the efficiency and success rate of the operation, and reduce the patient's pain.

In order to enable the anchoring section 110 to be accurately placed at the native valve annulus, the inner diameter of the first cavity 111 generally is comparable to the size of the native valve annulus. For example, in this embodiment, the inner diameter of the first cavity 111 is 35 mm ~ 55mm, so that the outer diameter of the anchoring section 110 can be greater than or equal to the inner diameter of the native valve annulus, and the anchoring section 110 can better anchor and seal at the native valve annulus. This application does not limit the shape of the anchoring section 110. It is preferred that the outer contour shape of the expanded anchoring section 110 is comparable to the inner contour shape of the native valve annulus to achieve better anchoring. For example, the expanded anchoring section 110 may be shaped as a circle or oval.

This application does not specifically limit the outer contour shape of the elastic wire 121 along the extension direction thereof. At least part of the outer contour shape of the elastic wire 121 along the extension direction thereof may be a curve. That is, the outer contour shape of the elastic wire 121 along the extension direction thereof may be a curve, or may be formed by connecting curves and straight lines. Preferably, at least part of the outer contour of the elastic wire 121 along the extending direction thereof may be arc-shaped or wavy. Continuing to refer to Fig. 1, in this embodiment, the elastic wire 121 in the supporting section 120 is arc-shaped, so that the elastic wire 121 can better fit into the predetermined chamber to increase the anchoring force, and will not damage myocardial tissue. Of course, in other embodiments, the elastic wire 121 in the supporting section 120 can also be wavy, such as S-shaped or non-S-shaped.

Preferably, the anchoring device 100 further includes a fixing part 130 connected to the end of at least some of the elastic wires 121 away from the anchoring section 110, that is, the fixing part 130 can be connected to all the elastic wires 121, or only to one or more of the elastic wires 121, and the fixing part 130 is configured to be arranged in the predetermined chamber. With such a structure, the elastic wires 121 connected to the fixing part 130 can not deform and move during the cardiac cycle, or only cause small deformation and movement, so that the elastic wires 121 connected to the fixing part 130 can always apply elastic force to the predetermined chamber, which ensures that the supporting section 120 can always provide stable fixation and support for the anchoring section 110 during the cardiac cycle. This can prevent the anchoring section 110 from moving inside the heart and causing failure of the operation. In addition, since the fixing part 130 can be connected to the end of the elastic wire 121 away from the anchoring section 110, it can prevent the end of the elastic wire 121 from directly contacting the inner wall of the predetermined chamber and scratching the inner wall of the predetermined chamber, further ensuring that the safety and reliability of implantation of the anchoring device 100. Of course, in other embodiments, the anchoring device 100 for prosthetic heart valve may not be provided with a fixing part, and the ends of all the elastic wires 121 away from the anchoring section 110 extend freely.

In this embodiment, the fixing part 130 is a fixing ring. After the anchoring device 100 is expanded, the inner diameter of the fixing ring may be less than, equal to, or greater than the inner diameter of the first cavity 111 formed after the anchoring section 110 is expanded. Preferably, the inner diameter of the fixing part 130 is smaller than the inner diameter of the first cavity 111 after the anchoring section 110 is expanded. The fixing part 130 thus configured will not hinder the periodic movement of the heart. In other embodiments, the fixing part 130 may have a non-annular structure, for example, may be implemented as a fixing block connected to at least part of the elastic wire 121 or other structures.

### EMBODIMENT 2

For the sake of clarity, the anchoring device 200 in Embodiment 2 is designated with a different reference number from the anchoring device 100 in Embodiment 1. However, even if the reference numbers are different, for the same parts, the description of the anchoring device 100 in Embodiment 1 is also applicable to the anchoring device 200 in Embodiment 2. The following mainly describes only the differences from Embodiment 1. For the same parts, please refer to Embodiment 1.

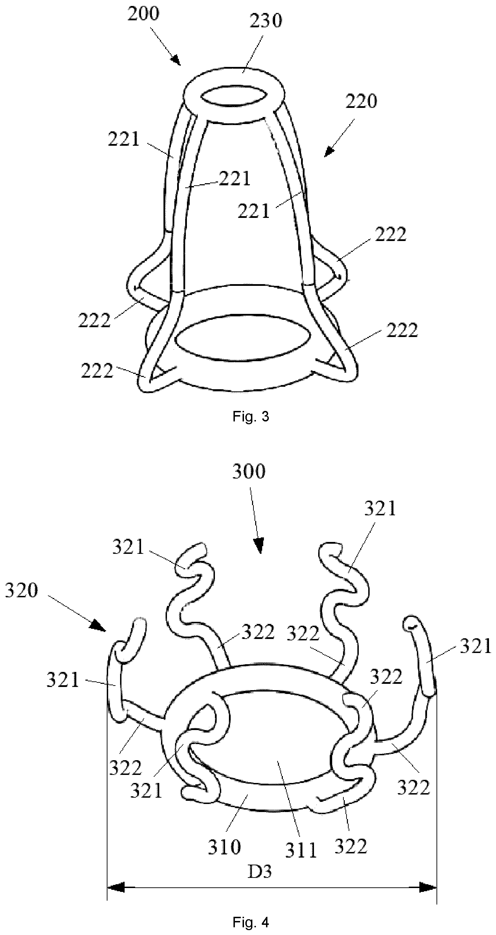

Referring to Figs. 2 and 3, the preferred Embodiment 2 of the present invention provides an anchoring device 200 for prosthetic heart valve. The anchoring device 200 includes an anchoring section 210 and a supporting section 220 that are axially connected to each other. The anchoring section 210 has an annular first cavity 211 capable of accommodating a prosthetic heart valve. The supporting section 220 is provided on one side of the anchoring section 210 along the axial direction. The supporting section 220 includes at least two elastic wires 221 arranged sequentially on the circumference of the anchoring section 210. One end of each elastic wire 221 is connected to the anchoring section 210, and the other end extends away from the anchoring section 210. Preferably, the anchoring device 200 further includes a fixing part 230. Please refer to Embodiment 1 for the implementation of the fixing part 230, which will not be described again here.

The anchoring device 200 of this embodiment is more elongated than the anchoring device 100 of the first embodiment, that is, the axial height H of the anchoring device after expansion is larger. It is configured in such a way that in the axial direction of the anchoring device 200, the axial height H of the supporting section 220 after expansion is greater than the axial height of the predetermined chamber, that is, the supporting section 220 has an interference fit with the predetermined chamber in the axial direction. At this time, since the anchoring section 210 abuts against the native annulus and cannot move further, so that the fixing part 230 can exert an elastic force on the predetermined chamber in the axial direction. Therefore, the supporting section 220 can be more firmly connected to the predetermined cavity, and it is ensured that the anchoring section 210 can be more firmly fixed at the desired position.

Referring to Fig. 2, in the radial direction of the anchoring device 200, the maximum outer contour of the expanded supporting section 220 in this embodiment may not contact the predetermined chamber and not form an interference fit. Therefore, the maximum outer diameter D2 of the expanded supporting section 220 is not limited. At this time, if the elastic wire 221 is arc-shaped, the bending radius may be the same as or different from the bending radius of the elastic wire 121 of Embodiment 1. It is preferred that the bending radius of the elastic wire 221 in this embodiment is smaller. In this way, during the cardiac cycle, the elastic wire 221 will have less interference or obstruction to the movement of the heart, or the elastic wire 221 will not have interference or obstruction to the movement of the heart. This can ensure the normal periodic movement of the heart. Moreover, the elastic wire 221 will not be deformed and moved due to the movement of the heart, further ensuring the anchoring force of the anchoring device 200. In other embodiments, in the radial direction of the anchoring section 210, the maximum outer contour of the expanded supporting section 220 in this embodiment may contact the predetermined chamber to form an interference fit. In this case, the bending radius of the elastic wire 221 is relatively large.

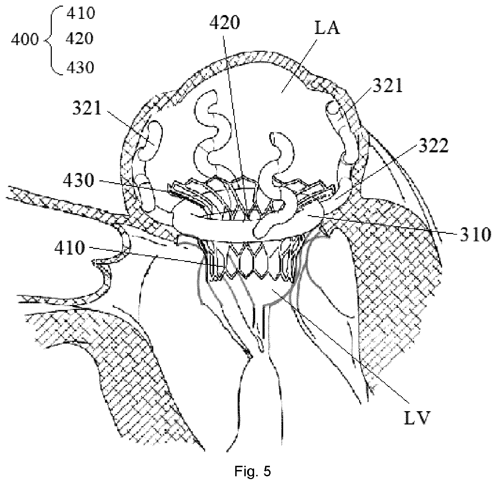

Referring to Fig. 5, an embodiment of the present invention also provides a prosthetic heart valve 400. The prosthetic heart valve 400 may adopt an existing structure. Specifically, it includes an outflow section 410 and an annulus section 420 that are connected with each other. In some cases, the prosthetic heart valve also includes the flange section 430, the outflow section 410, the annulus section 420 and the flange section 430 that are connected axially in sequence, and after the prosthetic heart valve is expanded, the inner diameter of the flange section 430 is larger than the inner diameter of the annulus section 420. However, this application does not limit the shape of the prosthetic heart valve 400. The shape of the prosthetic heart valve 400 can be cylindrical, conical, or any shape with a waist-shaped design.

In order to enable the prosthetic heart valve 400 with the flange section 430 to be implanted in the anchoring device 200, as shown in Fig. 3, the elastic wire 221 of this embodiment includes an avoidance section 222. The avoidance sections 222 on all the elastic wires 221 jointly define a second cavity (not labeled), which is configured to accommodate at least part of the prosthetic heart valve 400 (for example, the flange section 430 that can be used to accommodate the prosthetic heart valve 400). After the anchoring device 200 is expanded, the inner diameter of the second cavity is larger than the inner diameter of the first cavity 211 to prevent the anchoring device 200 from interfering with the flange section 430 of the prosthetic heart valve 400 and causing damage or movement of the prosthetic heart valve 400, thus avoiding the failure of the operation and danger to the patient's life safety. Of course, when the implanted prosthetic heart valve 400 does not include the flange section 430, the avoidance section 222 may not be provided on the elastic wire 221.

### EMBODIMENT 3

For the sake of clarity, the anchoring device 300 in Embodiment 3 is designated with a different reference number from the anchoring device 100 in Embodiment 1. However, even if the reference numbers are different, for the same parts, the description of the anchoring device 100 in Embodiment 1 is also applicable to the anchoring device 300 in Embodiment 3. The following mainly describes only the differences from Embodiment 1. For the same parts, please refer to Embodiment 1.

Referring to Fig. 4, a preferred embodiment of the present invention provides an anchoring device 300 for prosthetic heart valve. The anchoring device 300 includes an anchoring section 310 and a supporting section 320 that are axially connected to each other. The anchoring section 310 has a first cavity 311 capable of accommodating a prosthetic heart valve. The supporting section 320 is arranged on one side of the supporting section 310 along the axial direction. The supporting section 320 includes at least two elastic wires 321 arranged sequentially on the circumference of the anchoring section 310. One end of each elastic wire 321 is connected to the anchoring section 310, and the other end extends away from the anchoring section 310.

Different from Embodiment 1, the supporting section 320 of this embodiment includes six elastic wires 321 arranged symmetrically about the axis of the anchoring section 310 to achieve automatic positioning after the anchoring device 300 is released. Compared with Embodiment 1 in which the number of elastic wires 121 is four, a larger number of elastic wires 121 can further enhance the anchoring force of the supporting section 320.

Different from Embodiment 1, the shape of the elastic wire 321 in this embodiment is wavy, such as an S shape, so that the supporting section 320 can have a larger contact area when it contacts the predetermined chamber, thereby allowing the supporting section 320 to be firmly connected to the predetermined chamber, and better supporting and fixing the anchoring section 310. In this application, the number of times the elastic wire 321 is bent is not limited. The elastic wire 321 can be bent any number of times before being connected to the anchoring section 310.

Referring to Fig. 4, in this embodiment, in the radial direction of the anchoring device 300, the maximum outer diameter D3 of all supporting sections 320 after expansion is greater than the inner diameter of the contact portion of the predetermined chamber to the supporting section 320. In this way, the supporting section 320 is in interference fit with the predetermined chamber in the radial direction, thereby further allowing the supporting section 320 to be more firmly connected to the predetermined chamber. In addition, in this embodiment, the outer contour formed by the elastic wire 321 in the supporting section 320 may be spherical or cylindrical. It is preferably spherical, so that the outer contour formed by the elastic wires 321 can better approximate the shape of the predetermined chamber, and each elastic wire 321 in the supporting section 320 can contact the predetermined chamber to exert elastic force on the predetermined chamber.

Continuing to refer to Fig. 4, the differences from Embodiment 1 lie in that the elastic wires 321 in the supporting section 320 do not intersect but extend independently, and there is no fixing part on the top of the elastic wires 321, so that each elastic wire 321 can move independently along with the periodic movement of the heart when contacting with the predetermined chamber. In this way, the elastic wire 321 can better adapt to various shapes of the predetermined chamber and ensure that the supporting section 320 can always be firmly connected to the predetermined chamber. In addition, the design without a fixing part at the end of the elastic wire 321 is also conducive to reducing the overall height of the anchoring device 300, thereby reducing the size of the anchoring device 300 after being pressed, and simplifying the delivery device and reducing the difficulty of implantation of the anchoring device 300.

Referring to Figs. 4 and 5, in a specific embodiment, in order to enable the prosthetic heart valve 400 with the flange section 430 to be implanted in the anchoring device 300, each elastic wire 321 of the supporting section 320 of this embodiment includes avoidance sections 322. All the avoidance sections 322 on the elastic wires 321 jointly define a second cavity (not labeled), which is used to accommodate at least part of the prosthetic heart valve 400 (for example, it can be used to accommodate the flange section 430 of the prosthetic heart valve 400). After the anchoring device 300 is expanded, the inner diameter of the second cavity is larger than the inner diameter of the first cavity 311, and the presence of the avoidance section 322 can avoid interference between the anchoring device 300 and the flange section 430 of the prosthetic heart valve 400, which may cause damage or movement of the prosthetic heart valve 400. Of course, when the implanted prosthetic heart valve 400 does not include the flange section 430, the avoidance section 322 may not be provided on the elastic wire 321.

The preferred Embodiment 3 of the present invention also provides a method for delivering an anchoring device for prosthetic heart valve. The anchoring device 300 can be operated in a contracted state and an expanded state. The method for delivering the anchoring device 300 includes:
Contracting the anchoring device 300 in an external device so that the contracted anchoring device 300 can enter the predetermined chamber together with the external device; releasing and expanding the anchoring section 310; moving the external device so as to move the expanded anchoring section 310 to a predetermined position; releasing and expanding the supporting section 320 so that each elastic wire 321 in the expanded supporting section 320 can exert elastic force on the inner wall of the predetermined chamber.

Furthermore, the preferred Embodiment 3 of the present invention also provides a method for delivering a prosthetic heart valve system. The method for delivering the anchoring device 300 is as described above, wherein the prosthetic heart valve can be operated in the contracted state and the expanded state. The method of delivering a prosthetic heart valve includes:
After the anchoring device 300 is completely released, contracting the prosthetic heart valve in the external device and moving it with the external device to the first cavity 311 of the anchoring device 300; releasing the prosthetic heart valve so that the prosthetic heart valve expands in the radial direction, and the anchoring section 310 can exert an anchoring force on the expanded prosthetic heart valve to anchor the prosthetic heart valve at a predetermined position. It should be understood that the predetermined position in the present invention refers to the position where the anchoring section 310 is implanted. In this embodiment, the predetermined position refers to the position where the native valve annulus is located. The external device in the present invention refers to the device capable of carrying the compressed anchoring device and moving within a predetermined object (such as a human heart). In this embodiment, the external device refers to an anchoring device or a delivery system for a prosthetic heart valve.

Referring to Fig. 5, in this embodiment, the anchoring device 300 can be implanted into the left atrium LA, and the prosthetic heart valve 400 can be released into the first cavity 311 of the anchoring device 300 to replace and substitute the natural mitral valve.

The following takes mitral valve replacement surgery as an example to illustrate the implantation process of the anchoring device 300 and the prosthetic heart valve 400.

Referring to Fig. 5, the anchoring device 300 can be delivered by the delivery system into the left atrium LA via the interatrial septum or the cardiac apex. After implantation of the anchoring device 300, the anchoring section 310 and the supporting section 320 of the anchoring device 300 can be released at any position in the left atrium LA. Since the supporting section 320 is arranged symmetrically with respect to the axis of the anchoring section 310, the anchoring section 310 can automatically move to the native valve annulus after being released, and each elastic wire 321 of the supporting section 320 can automatically contact the inner wall of the left atrium LA and can exert elastic force on the left atrium LA. After the supporting section 320 is completely released, the operator can withdraw the delivery system to complete the implantation of the anchoring device 300. After the implantation of the anchoring device 300 is completed, the implantation of the prosthetic heart valve 400 begins. In Fig. 5, the prosthetic heart valve 400 includes an outflow section 410, an annulus section 420 and a flange section 430 that are connected axially in sequence. After the prosthetic heart valve 400 is expanded, the inner diameters of the outflow section 410 and the flange section 430 are both larger than the inner diameter of the annulus section 420. When the prosthetic heart valve 400 is implanted, the prosthetic heart valve 400 can be delivered by the delivery system into the left atrium LA through the interatrial septum, inferior vena cava, cardiac apex or other routes, and the outflow section 410 is placed in the left ventricle LV, and the flange section 430 is placed in the left atrium LA, and the annulus section 420 is placed within the first cavity 311 of the anchoring section 310. After the prosthetic heart valve 400 is implanted, the diameter of the prosthetic heart valve 400 is enlarged through one-time expansion by a balloon or self-expanding due to sheath withdrawal. During the diameter enlarging, the anchoring section 310 in the anchoring device 300 will undergo elastic deformation or plastic deformation. When the anchoring section 310 is deformed, the anchoring section 310 can adapt to the shape of the annulus section 420 in the prosthetic heart valve 400, and be fully fixedly connected and sealed with the annulus section 420, so that the anchoring device 300 can be fixedly connected to the prosthetic heart valve 400 as a whole, and the prosthetic heart valve 400 is anchored at the native valve annulus by the anchoring device 300.

In addition, the inner diameters of the outflow section 410 and the flange section 430 are both larger than the inner diameter of the annulus section 420. After the diameter of the prosthetic heart valve 400 is enlarged, the flange section 430 can be located in the second cavity of the anchoring device 300 in the left atrium LA, the outflow section 410 can be located in the left ventricle LV. At this time, the outflow section 410 and the flange section 430 with larger radii can limit the annulus section 420 of the prosthetic heart valve 400 in the axial direction, enabling the annulus section 420 to be located only at the anchoring section 310, thereby ensuring that the prosthetic heart valve 400 cannot move in the axial direction after implantation. After the prosthetic heart valve 400 is fully expanded, the operator can withdraw the delivery system after confirming that the prosthetic heart valve 400 and the anchoring device 300 have been firmly implanted in the desired position, thus completing the implantation operation of the prosthetic heart valve 400 and the anchoring device 300.

It should also be understood that the implantation process of the anchoring device 100 in Embodiment 1 of the present invention, the anchoring device 200 and the prosthetic heart valve 400 in Embodiment 2 of the present invention can be understood by referring to Embodiment 3, and will not be described in detail. In addition, the prosthetic heart valve system provided by embodiments of the present invention may include the anchoring device provided in any embodiment.

In conclusion, according to the anchoring device and the prosthetic heart valve system in the present invention, the prosthetic heart valve can be fixed at the heart valve annulus by the anchoring device. In specific implementation, the anchoring section of the anchoring device is provided at the native valve annulus, and the anchoring section is fixed by virtue of anchoring of the supporting section in the atrium or ventricle. In this way, since the supporting section is only provided on one side of the anchoring section along the axial direction, the supporting section does not need to cross the native valve annulus, thereby effectively preventing the occurrence of paravalvular leakage. In addition, this also avoids damage to the native valve leaflets or compression of the native valve annulus tissue. The operation is safe and reliable, and is beneficial to the patient's postoperative recovery.

The anchoring device of prosthetic heart valve provided by the present invention achieves anchoring only through the interference fit between the supporting section and the atrium or ventricle due to the elasticity of the supporting section, and the prosthetic heart valve is connected to the anchoring section by the interference fit between the first cavity of the anchoring section and the prosthetic heart valve, which results in a simple structure. When the anchoring device is released at any position in the atrium or ventricle during the implantation, the anchoring section can be moved automatically to the desired position, which can simplify the implantation process of the anchoring device, reduce the difficulty of implanting the anchoring device and improve the reliability and convenience of prosthetic heart valve surgery.

The above description is only for the description of preferred embodiments of the present invention, and is not intended to limit the scope of the present invention. Any changes and modifications made by those skilled in the art according to the above disclosure are all within the protection scope of the appended claims.

## Claims

1. An anchoring device for prosthetic heart valve, comprising an anchoring section and a supporting section that are axially connected to each other; wherein the anchoring section has an annular first cavity configured to accommodate a prosthetic heart valve, and the anchoring section is configured to self-adapt to an outer contour of the prosthetic heart valve when being deformed by a force so as to exert anchoring force on the prosthetic heart valve;
the supporting section is arranged on one side of the anchoring section along an axial direction thereof, and includes at least two elastic wires arranged sequentially on a circumference of the anchoring section; each of the elastic wires has an end connected to the anchoring section, and has a further end extending in a direction away from the anchoring section;
the anchoring device is configured such that the anchoring section is configured to be arranged at annulus of a predetermined object, and the supporting section is configured to be arranged in a predetermined chamber of the predetermined object and can be fixed in the predetermined chamber by elastic force.

2. The anchoring device for prosthetic heart valve according to claim 1, wherein in a radial direction of the anchoring device, a maximum outer diameter of the supporting section after expansion is greater than an inner diameter of a contact portion of the predetermined chamber to the supporting section, and/or, in an axial direction of the anchoring device, an axial height of the supporting section after expansion is greater than an axial height of the predetermined chamber.

3. The anchoring device for prosthetic heart valve according to claim 1, wherein a partial contour of the elastic wire along an extension direction thereof is curved.

4. The anchoring device for prosthetic heart valve according to claim 3, wherein the partial contour of the elastic wire along the extension direction thereof is arc-shaped or wavy.

5. The anchoring device for prosthetic heart valve according to claim 3, wherein each of the elastic wires comprises an avoidance section, and wherein the avoidance sections on all the elastic wires jointly define a second cavity configured to accommodate at least part of the prosthetic heart valve; after the anchoring device is expanded, an inner diameter of the second cavity is larger than an inner diameter of the first cavity.

6. The anchoring device for prosthetic heart valve according to claim 1, further comprising a fixing part connected to the further end of one or more of the elastic wires, and wherein the fixing part is arranged in the predetermined chamber.

7. The anchoring device for prosthetic heart valve according to claim 6, wherein the fixing part is a fixing ring, and an inner diameter of the fixing ring is smaller than an inner diameter of the first cavity after the anchoring device is expanded.

8. The anchoring device for prosthetic heart valve according to any one of claims 1 to 7, wherein all the elastic wires are evenly distributed on the circumference of the anchoring section.

9. The anchoring device for prosthetic heart valve according to any one of claims 1 to 7, wherein the anchoring section is structured as a coil formed by winding wire material, and the coil has at least one turn.

10. A prosthetic heart valve system, comprising a prosthetic heart valve and the anchoring device for prosthetic heart valve according to any one of claims 1 to 9, wherein the prosthetic heart valve is configured to be accommodated in the anchoring device and fixed in the predetermined object by the anchoring device.
